# EUROPEAN PATENT SPECIFICATION

(11) **EP 4 329 594 B1**
(45) Date of publication and mention of the grant of the patent: **21.10.2026**
(21) Application number: 22725423.2
(22) Date of filing: 25.04.2022
(51) Int. Cl.: A61B 5/00, A61B 5/1455, G01S 7/481, G01S 7/484, G01S 7/4861, G01S 7/4863, G01S 17/10, G01S 17/89, A61B 5/026, A61B 5/024

(54) **OPTOELECTRONIC APPARATUS FOR AND METHOD OF MEASURING ORGANIC TISSUE**
OPTOELEKTRONISCHE VORRICHTUNG UND VERFAHREN ZUR MESSUNG VON ORGANISCHEM GEWEBE
APPAREIL OPTOÉLECTRONIQUE ET PROCÉDÉ DE MESURE DE TISSU ORGANIQUE

(30) Priority: 27.04.2021 FI 20215487
(43) Date of publication of application: 06.03.2024
(73) Proprietor: Oulun yliopisto, 90570 Oulu (FI)
(72) Inventor: NISSINEN, Jan, 90570 Oulu (FI)
(74) Representative: Kolster Oy Ab
(86) International application number: PCT/EP2022/060853
(87) International publication number: WO 2022/229073

(56) References cited:
- WO-A1-2020/010276
- WO-A1-2020/243161
- US-A1- 2016 007 864
- US-A1- 2018 333 060
- US-A1- 2019 239 753
- US-A1- 2020 352 445
- US-A1- 2021 026 011

## Description

### Field

The invention relates to an optoelectronic apparatus for and a method of measuring organic tissue.

### Background

Oxygen content variation of blood and pulse of the heart can be measured optically in a non-invasive manner using a transmissive oximeter. The oximeter attached to a fingertip or earlobe typically has a continuously illuminating LED (Light Emitting Diode), although a laser may also be used, that outputs infrared light, and the oximeter measures absorbance of the light that depends on oxygenation of haemoglobin. A reflectance oximeter performs the measurement basically in a similar manner to the transmissive measurement except that the light does not travel from one side of a section of body such as chest or arm to another side but instead light scattered and/or reflected back from the section of a body is measured. These measurements work, however, poorly for skin, muscles and venous blood, for example.

Patent document WO2020010276 teaches how to perform transabdominal fetal oximetry using optical tomography. Patent document US20190239753 presents an interleaved photon detection array for optically measuring a physical sample.

Time-domain near infrared spectroscopy, which is based on transmission and reception of short optical pulses and determination of temporal duration therebetween, has also been used for providing concentration values of oxygenation levels of haemoglobin. However, the devices are not fully developed. Hence, improvement in biological and/or bio-signal measurements performed in an optical manner would be welcome.

### Brief description

The present invention seeks to provide an improvement in the measurements.

The invention is defined by the independent claims. Embodiments are defined in the dependent claims.

### List of drawings

Example embodiments of the present invention are described below, by way of example only, with reference to the accompanying drawings, in which
Figure 1 illustrates an example of a time-of-flight measuring apparatus;
Figure 2 illustrates an example of a measurement of organic tissue;
Figure 3 illustrates an example of a measurement with optical cables;
Figure 4 illustrates an example of a coaxial measurement;
Figure 5 illustrates an example of an array of single-photon avalanche diodes (SPADs);
Figure 6 illustrates examples of output and receive optical pulses;
Figure 7 illustrates an example of separation of useful signal from disturbance;
Figure 8 illustrates an example of the SPADs a part of which is covered by a polarizer;
Figure 9 illustrates an example of a change of slopes of haemoglobin and lactate concentrations;
Figures 10 and 11 illustrate examples of the measuring apparatus of separate parts that communicate through radio transmission;
Figure 12 illustrates an example of a data processing unit;
Figure 13 illustrates an example of a single circuit board; and
Figure 14 illustrates of an example of a flow chart of a measuring method.

### Description of embodiments

The following embodiments are only examples. Although the specification may refer to "an" embodiment in several locations, this does not necessarily mean that each such reference is to the same embodiment(s), or that the feature only applies to a single embodiment. Single features of different embodiments may also be combined to provide other embodiments. Furthermore, words "comprising" and "including" should be understood as not limiting the described embodiments to consist of only those features that have been mentioned and such embodiments may also contain features/structures that have not been specifically mentioned. All combinations of the embodiments are considered possible if their combination does not lead to structural or logical contradiction.

It should be noted that while Figures illustrate various embodiments, they are simplified diagrams that only show some structures and/or functional entities. The connections shown in the Figures may refer to logical or physical connections. It is apparent to a person skilled in the art that the described apparatus may also comprise other functions and structures than those described in Figures and text. It should be appreciated that details of some functions, structures, and the signalling used for measurement and/or controlling are irrelevant to the actual invention. Therefore, they need not be discussed in more detail here.

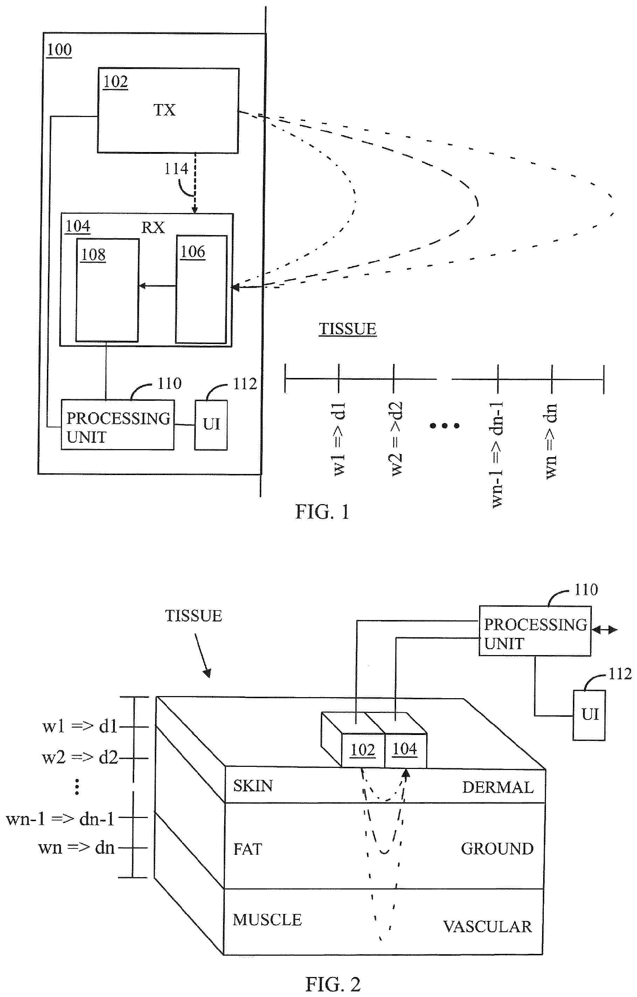

Figure 1 illustrates an example of an optoelectronic apparatus 100 utilizing time-of-flights of photons of optical pulses. A semiconductor optic radiation source 102 outputs repeatedly infrared pulses. In an embodiment, the semiconductor optic radiation source 102 may comprise a CMOS (Complementary Metal Oxide Semiconductor) radiation source. The optic radiation source 102 may comprise one or more optically radiating semiconductor lasers. A plurality of the semiconductor lasers may be in a form of an array.

In an embodiment, duration of the optical pulses may be less than about 1 ns, for example. In an embodiment, duration of the optical pulses may be less than about 500 ps, for example.

The repetition of the optical pulses may be regular or irregular. The repetition may have a certain frequency, for example. The repetition rate may be adjustable.

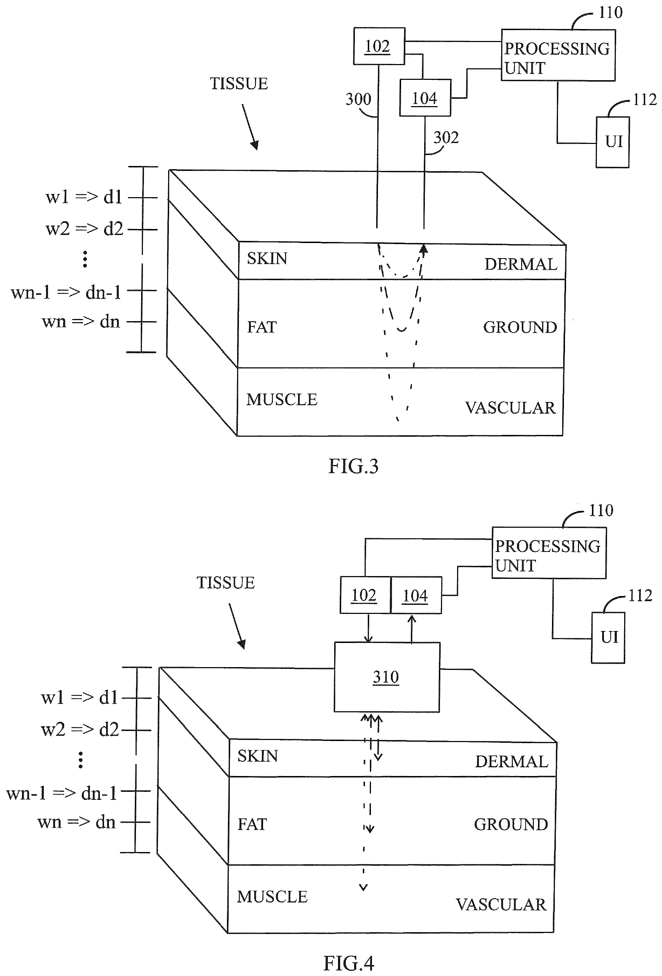

The optical pulses are directed toward an organic tissue, which is illustrated in more detail in Figures 2, 3 and 4. In an embodiment, the organic tissue may be a piece of meat, for example. The organic tissue may be living tissue. In an embodiment, the organic tissue may be that of an animal, for example. In an embodiment, the organic tissue may be that of a mammal such as a human being, for example. The organic tissue of a living animal can be considered to have three basic layers from surface towards inside: skin, fat and muscle as illustrated in Figures 2 and 3. In an embodiment, the optoelectronic apparatus 100 may be worn by the animal such as the human being.

In an embodiment, the organic tissue may be that of a plant such as a tree, grass or moss, for example. The organic tissue of a plant can be considered to have three basic layer from surface towards inside: dermal, ground and vascular as illustrated in Figures 2, 3 and 4. However, the number of layers is may be different or the layers may be unimportant for the measurement presented in this document.

The organic tissue may include one organ or a plurality of organs. Tissue of a single organ is a community of similar cells that function in similar manner. Blood is both tissue of cells and flowable substance. The physiological state of blood may refer to a level of haemoglobin, tendency of the level of haemoglobin and/or difference between oxidized haemoglobin and deoxidized haemoglobin. Additionally or alternatively the physiological state of blood may refer to variation of blood volume as a function of time in the tissue i.e. pulsation of the heart. Correspondingly, a physiological state of a plant tissue may depend on at least one flowable substance therewithin.

An array 106 of single-photon avalanche diodes 400 of an optical receiver 104, an example of which is illustrated in Figure 4, is also directed toward the tissue. That the single-photon avalanche diodes 400 are directed toward the organic tissue means that field-of-views 402 of the single-photon avalanche diodes 400 are directed toward the organic tissue. The single-photon avalanche diodes 400 are configured to detect photons of the optical pulses that have interacted with the organic tissue.

The single-photon avalanche diodes (SPADs) 400 of the optical receiver 104 operate in the Geiger mode and not in a linear mode. Hence, the output of the single-photon avalanche diodes 400 is binary indicating only a detection or nondetection, the detection being triggered by a single photon.

Figure 2 illustrates an example of a measurement where the radiation source 102 and the optical receiver 104 are attached with the skin or the dermal layer.

Figure 3 illustrates an example where optical transmission cable 300 and optical reception cable 302 are used. The optical cables 300, 302 may comprise one or more optical fibers. In Figure 3, the optical cables 300, 302 are side by side but they could also be coaxial. It is also possible that only the radiation source 102 has the optical cable 300 and the optical receiver 104 without the optical receiving cable 302 is in contact with the skin or the dermal layer. Alternatively, it is possible that only the optical receiver 104 has the optical receiving cable 302 and the radiation source 102 without the optical transmission cable 300 is in contact with the skin or the dermal layer.

A timing unit 108, which comprise an electric circuit, determines a time interval between an output of an optical pulse and reception of each of the photons of the optical pulse from the tissue. In this application, the term "determine" in its various grammatical forms may mean calculating, computing, data processing for deriving a result, looking up in a database or the like. As a result "determine" may also mean select, choose or the like.

The optical pulse transmitted from the the radiation source 102 may travel along various paths through the tissue, which are illustrated with dashed lines in Figure 1 to 4. The the radiation source 102 and the optical receiver 104 may be connected such that with an optical pulse transmitted towards the tissue, a reference signal 114 is simultaneously transmitted to the optical receiver 104 for determining an output moment of an optical pulse. A portion of the optical pulse may be transmitted to the optical receiver 104 via a partially reflective mirror or prism or the like as the reference signal 114 for determining the output moment of an optical pulse, while a major portion of the optical pulse is transmitted towards the tissue. Alternatively, the the radiation source 102 may directly transmit another electrical or optical pulse as the reference signal 114 to the optical receiver 104 for determining the output moment. In general, the reference signal 114 has a determined temporal dependence with respect to the optical pulse transmitted towards the tissue for determining the time-of-flights.

A portion of the photons of an output optical pulse 500 reflect and/or scatter from the tissue and hit the array 106 of the single-photon detectors 400. In any of the single-photon detectors 400, the photon may be detected as a result of a high-speed breakdown in the detector 400. The timing jitter of the detection may be in a CMOS single-photon detector at the level of about 10 ps to 50 ps. Moreover, the breakdown may introduce immediately a logic level signal (e.g. 3 V) and thus no analog amplifiers may be needed. In addition to the 2D array 106 of the SPAD detectors 400, the optical receiver 104 may also include the timing unit 108, which may have a plurality of time-to-digital converters (TDCs). In general, the timing unit 108 may be a part of the optical receiver 104 or it may be outside of the optical receiver 104.

In an embodiment, the semiconductor optic radiation source 102 may enable the array 106 of single-photon avalanche diodes 400 for detections but it may be the array 106 of single-photon avalanche diodes 400 that controls the time-to-digital converters. That is, the time-to-digital converter consumes electric energy only in conjunction with the detections of photons at the single-photon avalanche diodes 400 which save energy in general. The length of the temporal measurement range 510 may be determined using a delay from the transmission of the optical pulse. After this determined delay detections are stopped. The temporal measurement range 510 may be based on a temporal range of the time-to-digital converter.

Time-to-digital conversion may thus be realized with about 10 ps to 50 ps resolution, for example, and it may measure the arrival-times of detected photons for reconstructing distribution of time of flight of photons.

The functionality of the time-to-digital converters is thus to measure the interval between the emitted laser pulse 500 and the introduced breakdown in every SPAD detector 400 that detected a photon. These intervals are the transit times of the photons from the radiation source 102 to the optical receiver 104 via the organic tissue.

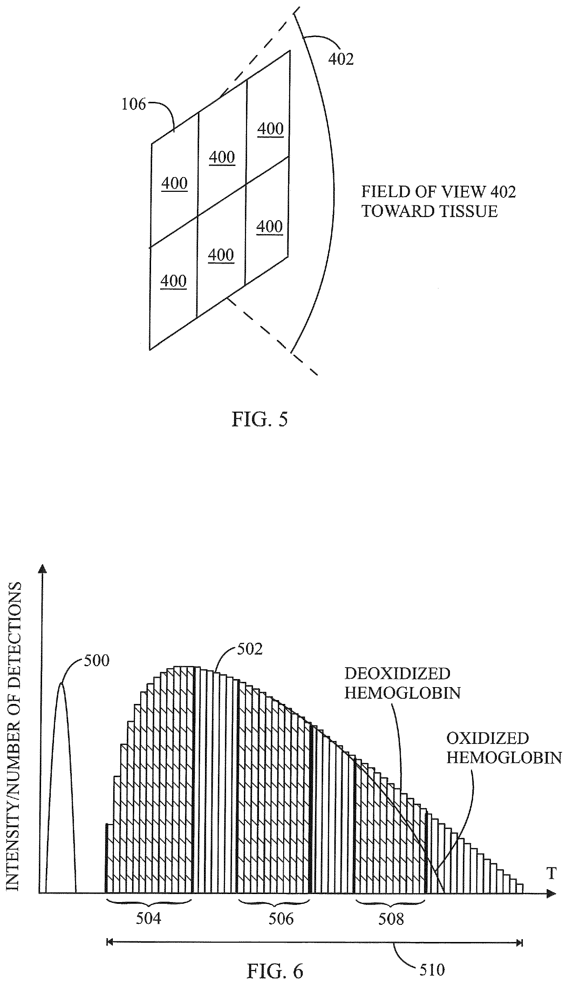

Figure 5 illustrates examples of the output optical pulse 500 and the received optical pulse 502 interacted with the organic tissue. The x-axis is time T in an arbitrary scale and the y-axis is intensity and number of detections in arbitrary scales. The timing unit 108 determines time-of-flights of photons of each of the optical pulses within a temporal measurement range 510 after an output of each of the optical pulses. The temporal range 510, which is an allowed range within which time-of-flights of photons are measured, may start after a certain dead-time from an output of an optical pulse such that a reflection from the optical components of the apparatus, hair(s), other extension and/or liquid(s) layer or drops on the outer surface and/or the outer surface of the tissue itself are cut out from the measurement, for example. Each of the time-of-flights is based on an output moment of an optical pulse from the semiconductor optic radiation source 102 and detection moments of photons of the optical pulse 502 by the single-photon avalanche diodes 400 of the array 106.

Because of scattering in the tissue the received optical pulse 502 is much wider than the output optical pulse 500. A data processing unit 110 estimates or determines a physiological state of the tissue within at least one time window 504, 506, 508 shorter than the temporal measurement range 510 based on at least one of the following: a number of the detections within each of the time window 504, 506, 508 and a distribution of the detections within at least one time window 504, 506, 508. In this manner, the organic tissue may be measured in a layered manner regardless layered or non-layered structure of the organic tissue. Here, the term "estimate" in its various grammatical forms may mean calculate, compute, data process, look up in a database or the like for at least approximate result.

The physiological state may define or estimate functions of the at least one organ or at least one part of the at least one organ of the organic tissue. A variety of changes in the organic tissue result in changes in transit times of photons which may make the shape of the optical pulse 502 interacted with the organic tissue depend on the change in the tissue. That is, the transit times of the photons are a function of a state of the organic tissue. The shape refers to distribution of detections over time. Alternatively or additionally, a number of detections at a certain moment or within a time window may vary as a function of state of the organic tissue.

In an embodiment, each of the single-photon avalanche diodes 400 may detect a single photon of each of the optical pulses that is scattered from the tissue toward the array 106.

In an embodiment, the array 106 of single-photon avalanche diodes 400 may detect at least one of the following: a number of detections of at least one wavelength dominantly absorbed by blood with deoxidized hemoglobin; and a number of detections of at least one wavelength dominantly absorbed by blood with oxidized hemoglobin. The deoxidized hemoglobin mainly resides in veins. The deoxidized hemoglobin is within blood that is non-saturated with oxygen. The oxidized hemoglobin mainly resides in arteries. The oxidized hemoglobin is within blood that is saturated with oxygen.

Figure 6 illustrates an example of how deoxidized hemoglobin and oxidized hemoglobin affect a shape of optical pulse and/or the number detections. The y-axis denotes the intensity of the optical pulse 500 and the number of detections of the received optical pulse 502 in arbitrary scales, and the x-axis denotes time T in an arbitrary scale. Figure 6 is partially a histogram where the received pulse 502 is illustrated as bars. In time scale, a width of each bar represents an integration period over which detections are counted for one optical pulse. For a more reliable measurement, a sum of the number of detections of a plurality of optical pulses may be counted for each bar (that has been done in the example of Figure 6). The integration period of a single bar may be determined be a temporal resolution of the time-to-digital conversion, for example. The integration period may thus be about 10 ps to 50 ps i.e. a temporal width of each of the bars, for example. However, some other integration period may also be used. Any of the bars alone may be used as a time window within which a measurement are performed. However, a measurement may be performed over a plurality of bars like in time windows 504, 506 and 508, for example.

The time window 504 may related to skin or dermal. The time window 506 may relate to fat or ground. The time window 508 where the time-of-flight is longer than in the time windows 504 and 506 can be considered to relate to muscle of an animal. The more oxidized the hemoglobin of blood of the muscle is, the lower is the number detections in the time window 508 relating to the muscle. The difference between the oxidized hemoglobin of blood of the muscle and the deoxidized hemoglobin of blood can also be seen the shape of the received optical pulse 502. Namely, the received optical pulse 502 is the shorter (see the continuous line), the more oxidized hemoglobin of blood is in the muscle. Corresponding changes may be observed in plants.

An optical photoplethysmogram (PPG) measurement can be used to detect variation of blood volume in the tissue based on changes in light absorption. In an embodiment of this document, the time-of-flights of photons of each of the optical pulses may be utilized to detect variation of blood volume such that a number of detections photon in at least one time window is determined. The number of detections is related to the absorption of the photoplethysmogram measurement. In an embodiment, a measurement rate of the variation of blood volume may be about ten measurements per second. In an embodiment, a measurement rate of the variation of blood volume may be about five measurements per second.

A received optical signal X_{dk}(t) as function of time t from a certain depth dk of the tissue may be expressed in a mathematical form in the following manner: ${X}_{dk} \left(t\right) = {H}_{dk} \left(t\right) + {D}_{dk} \left(t\right)$ where H_{dk}(t)is a temporal variation of strength i.e. number of detections caused by the variation of blood volume i.e. pulsation of heart in the depth dk, and D_{dk}(t) includes other temporal variation such as optical losses as a function of time and movement of the tissue which can be considered disturbance. The movement of the tissue may be caused by movement of body or limb, for example.

The received optical signal X_{dn}(t) as function of time from depths d1 to dn of the tissue may be expressed in a mathematical form in the form of group of equations: $\begin{matrix}{X}_{d 1} \left(t\right) = {H}_{d 1} \left(t\right) + {D}_{d 1} \left(t\right) \\ {X}_{d 2} \left(t\right) = {H}_{d 2} \left(t\right) + {D}_{d 2} \left(t\right) \\ ⋮ \\ {X}_{dn} \left(t\right) = {H}_{dn} \left(t\right) + {D}_{dn} \left(t\right)\end{matrix}$ which may be expressed as a matrix: ${X}_{d} \left(t\right) = {H}_{d} \left(t\right) + {D}_{d} \left(t\right)$ where **X**_{d}(t), **H**_{d}(t) and **D**_{d}(t) are time dependent matrices.

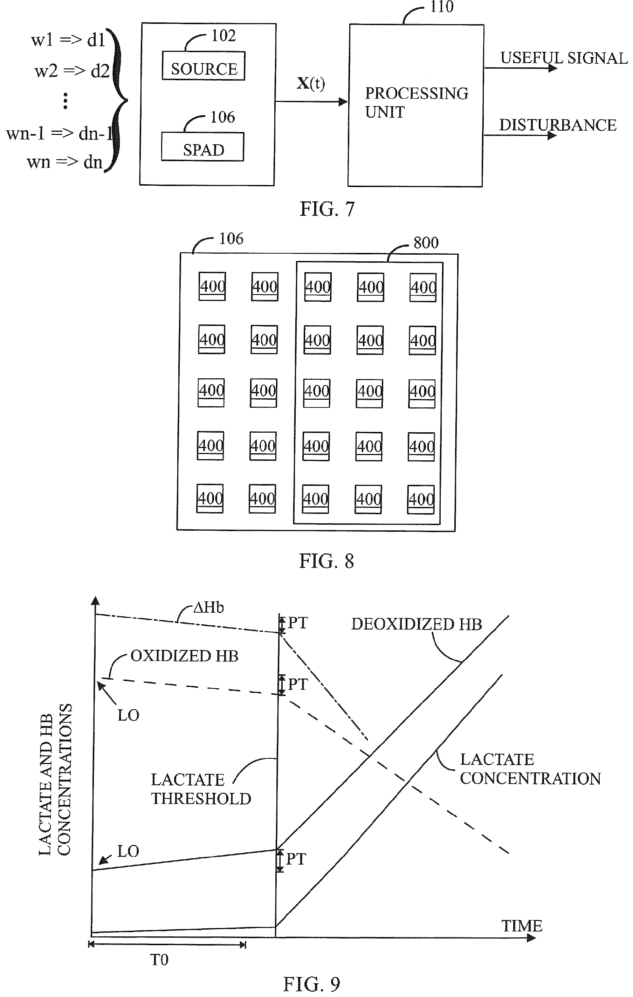

There is one to one correspondence between the depths d1 to dn and time windows w₁ to wₙ of measured time-of-flights. The time windows w₁ to wₙ can also be considered to be ranges of times of flights. That is, the data processing unit 110 may perform an estimation of the physiological state of the tissue within each of a plurality of the time windows as a function of time. The disturbance D_{dk}(t) varies as a function of depth and thus of the time window because different levels of the tissue respond differently to movement and contact variation, but the variation of blood volume i.e. each phase of the pulsation of heart occurs synchronously in all depths and time windows. As shown in Figure 7, this makes it is possible for the data processing unit 110 to cancel out or reduce the disturbance and to separate and determine the variation of blood volume i.e. pulsation of heart in a reliable manner also under disturbance based on the estimation. The pulsation of the heart which may also be called heartbeat may be separated from the received signal using principal component analysis (PCA), independent component analysis (ICA), Kalman filter, digital filter, neural network, artificial intelligence, machine learning or the like, for example. A person skilled in the art is familiar with disturbance cancellation, reduction and/or separation of a useful signal, *per se.*

As explained, the data processing unit 110 estimates the physiological state at at least one unique depth range 504, 506, 508, w1 to wn within the tissue using detected photons that have interacted with the tissue inside at least one time window 504, 506, 508, w1 to wn shorter than the temporal measurement range 510. Each of the at least one time window 504, 506, 508, w1 to wn corresponds to a unique depth range d1 to dn within the tissue. The data processing unit 110 can then estimate the physiological state at at least one unique depth range 504, 506, 508, w1 to wn based on a number of the detections within each of the at least one time window 504, 506, 508, w1 to wn. The data processing unit 110 can alternatively estimate the physiological state based on a distribution of the detections of a plurality of time windows 504, 506, 508, w1 to wn. Of course, a combination of these is also possible.

Figure 8 illustrates an example where a part of the SPADs 400 of the array 106 is covered by a polarizer 800. The polarizer 800 may be used to give more information on the tissue.

The data processing unit 110 may then estimate or determine physiological state related to lactic acid. In an embodiment, the data processing unit 110 may estimate or determine build-up of lactic acid in the tissue, for example.

In an embodiment, the data processing unit 110 may perform the estimation based on the number of detections of the at least one wavelength dominantly absorbed by the blood with oxidized hemoglobin. In an embodiment, the data processing unit 110 may perform the estimation based on difference between the number of detections of the at least one wavelength dominantly absorbed by the blood with deoxidized hemoglobin and the number of detections of the at least one wavelength dominantly absorbed by the blood with oxidized hemoglobin. In an embodiment, the data processing unit 110 may perform the estimation based on the number of detections of the at least one wavelength dominantly absorbed by the blood with oxidized hemoglobin, and difference between the a number of detections of the at least one wavelength dominantly absorbed by the blood with deoxidized hemoglobin and the number of detections of the at least one wavelength dominantly absorbed by the blood with oxidized hemoglobin. In an embodiment, at least one of these measurements may be performed from a muscle.

Figure 9 illustrates an example of tendency of how lactate concentration, oxidized haemoglobin and deoxidized haemoglobin concentrations behave in a muscle tissue during incremental exercise from rest to maximal load. The vertical axis denotes lactate and haemoglobin concentrations in arbitrary scale. The horizontal axis denotes time in arbitrary scale.

In order to delivery enough oxygen to the working muscles for the aerobic production of adenosine triphosphate (ATP) molecules in a high intensity activity, the respiratory rate of the athlete increases. If the load of the training is high, the oxygen delivery to the muscles is too slow to produce the adequate amount of energy the muscle requires. In that case the body's energy production shifts to the anaerobic condition at a threshold (see Figure 9). In this condition, energy production is replaced by lactic acid fermentation. Thus, measuring the oxidation of haemoglobin (oxidized haemoglobin Hb02 and/or deoxidized haemoglobin Hb) as explained above, the moment of the shift to anaerobic condition and the lactate threshold of the specific muscle can be determined.

During the incremental exercise, the oxidized haemoglobin Hb02 will decrease slowly before the time point of blood lactate threshold and then decrease. The decrease may be radical and potentially linear. Lactate of the blood increases slowly before the occurrence of lactate threshold and then increases. The increase may be sharp and potentially linear. Hence, there is a correlation between measured oxidized haemoglobin Hb02 and lactate threshold.

In an embodiment, approach to the turning point of lactate production i.e. the lactate threshold may be determined by evaluating the change in the slope of the oxidized haemoglobin Hb02 and/or the deoxidized haemoglobin Hb in blood. As the decrease of the oxidized haemoglobin Hb02 and/or the increase of the deoxidized haemoglobin Hb in blood has continued for a predetermined time T0 and/or change of the oxidized haemoglobin Hb02 and/or the deoxidized haemoglobin Hb with respect to the original level LO is equal to or larger than a predetermined change PT. In this manner, physical exercise may be lightened or stopped before the detection that the lactate production is at the lactate threshold. The predetermined time T0 and the predetermined change PT may be adaptive. The predetermined time T0 and the predetermined change PT may vary with respect to sex, exercise type, exercise length, age, physical condition, amount of physical training earlier or the like, for example.

In an embodiment, the turning point to the lactate production i.e. the lactate threshold may be determined by evaluating how rapid the change in the slope of the oxidized haemoglobin Hb02 and/or the deoxidized haemoglobin Hb in blood. In an embodiment, when the slope is different at different moments, the lactate threshold is between the moments of time. In an embodiment, when difference of the slope at different moments is larger than a predetermined value PV, the lactate threshold may be considered to be between said moments of time. In this manner, physical exercise may be lightened or stopped at the detection that the lactate production has reached at the lactate threshold. The predetermined value PV may vary with respect to sex, exercise type, exercise length, age, physical condition, amount of physical training earlier (professional, intensive amateur, normal amateur/exerciser, occasional exerciser, non-exerciser, for example) or the like, for example.

The difference ΔHb of the oxidized haemoglobin Hb02 and the deoxidized haemoglobin Hb also shows similar turning point and the variation tendency to lactate of the blood. Consistent with the physiology theory, the curve of difference ΔHb will show a fluctuation period around the analyzed oxygen dissociation point. Rightly in this fluctuation period, the metabolism status of the body is changed. The difference ΔHb may be a function of the oxidized haemoglobin Hb02 and the deoxidized haemoglobin Hb such as their difference. Alternatively or additionally, the difference ΔHb may be a function of a change of the oxidized haemoglobin Hb02 and a change of the deoxidized haemoglobin Hb.

In an embodiment, the data processing unit 110 may determine temporal variation of detections of a plurality of output optical pulses 500, the detections being within a window 506 of a middle part of the optical pulse 502 which is received after interaction with the tissue that is assumed fat tissue when examining an animal. The data processing unit 110 may then compensate the temporal variation of the detections in a time window 508 that has longer time-of-flights than the detections of the window 506 of the middle part of the optical pulse 502 based on the temporal variation of detections within the window 506 of the middle part of the optical pulse 502 which is received after interaction with the tissue that is fat tissue. The fat tissue has less circulation of blood than other tissues and the fat tissue is less prone to disturbances coming from the tissue related to the time window 508 deeper below the skin, which allows the data processing unit 110 to determine and reduce effect of noise and systematic disturbance.

Correspondingly, ground tissue of a plant may be used to determine temporal variation of detections of a plurality of output optical pulses 500. And in a similar manner the ground tissue of the plant is less prone to disturbances coming from the tissue related to the time window 508 deeper below the dermal, which allows the data processing unit 110 to determine and reduce effect of noise and systematic disturbance.

Noise and the systematic disturbance may be caused by movement of the animal or the plant.

In an embodiment, at least one of the timing unit 108 and data processing unit 110 may be programmable such that at least one of the temporal measurement range 510 and the at least one time window 504, 506, 508 is repeatedly adjustable. The at least one time window 504, 506, 508 may be adjusted manually or automatically. Manual adjustment may be performed through a user interface 112. In this manner, the organic tissue may be scanned in a depth direction, for example.

In an embodiment, the semiconductor optic radiation source 102 and the array 106 of single-photon avalanche diodes 400 may be spaced apart by a non-zero distance on the skin, an example of which is illustrated in Figure 2.

In an embodiment an example of which is illustrated in Figure 4, an output section 310 of the semiconductor optic radiation source 102 and an input section 310 of the array 104 of single-photon avalanche diodes 400 may be optically coaxial on the skin. In this embodiment, optical radiation hits the same area of the skin or the dermal both when entering the animal or the plant and when exiting the animal or the plant.

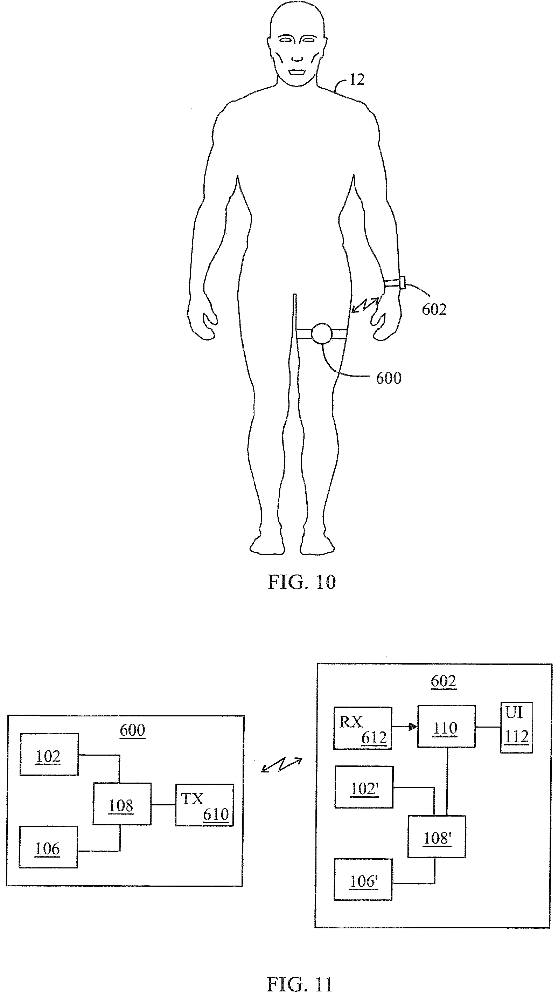

In an embodiment an example of which is illustrated in Figures 10 and 11, the optoelectronic apparatus 100 may comprise separately a muscle measurement device 600, which is wearable and attached to a muscular area of a body 12 of an animal such as a human being. The muscular area may be a thigh or upper arm, calf, neck, for example. The muscle measurement device 600 may comprise a radio transmitter 610, the semiconductor optic radiation source 102, the array 106 of single-photon avalanche diodes 400 and the timing unit 108. The optoelectronic apparatus may also comprise a separate wearable device 602, which comprises a radio receiver 612 and the data processing unit 110. The separate wearable device 602 may be attached at a wrist, for example. The radio transmitter 610 may transmit information on detections to the radio receiver 612, which may feed the information to the data processing unit 110 for determining physiological state of the tissue at the muscular area of the body 12. The user interface 112 may present the results to a user or a person or a personal performing the examination. The radio transmitter 610 and the radio receiver 612 may perform transmissions using electromagnetic radiation of radio frequency, Bluetooth^{®}, WLAN (Wireless Local Area Network) or the like.

In an embodiment, the separate wearable device 602 may comprise an additional semiconductor optic radiation source 102', and an additional array 106' of the single-photon avalanche diodes 400 for determining physiological state of the tissue at the wrist. The additional semiconductor optic radiation source 102', and the additional array 106' of the single-photon avalanche diodes 400 are similar to the semiconductor optic radiation source 102, and the array 106, respectively.

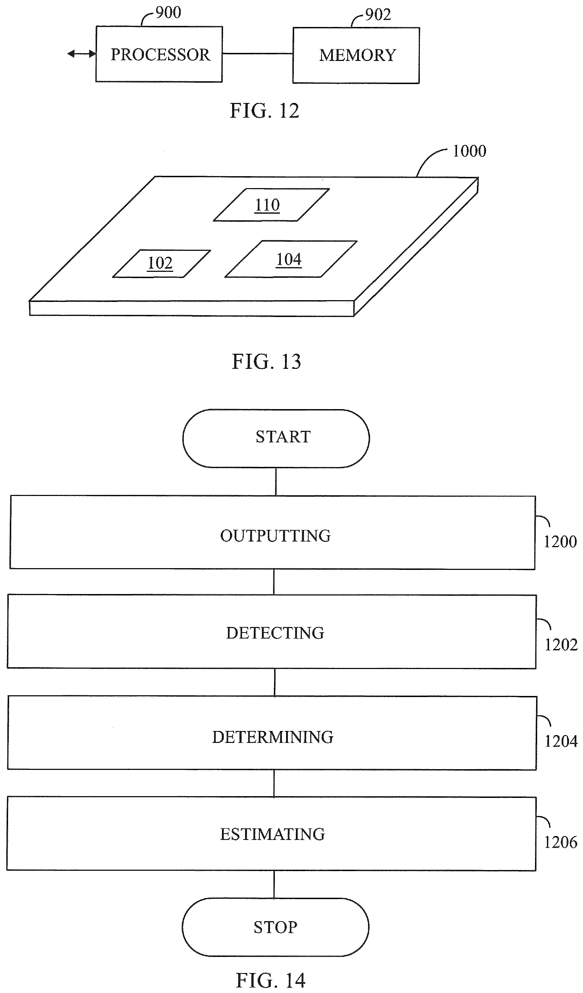

In an embodiment an example of which is illustrated in Figure 12, the data processing unit 110 may comprise one or more processors 900, and one or more memories 902 including computer program code. The one or more memories 902 and the computer program code may, with the one or more processors 900, cause the data processing unit 110 at least to estimate the physiological state of the tissue.

Figure 13 illustrates an example of a single circuit board 1000 the optoelectronic apparatus for measuring organic tissue. The circuit board 1000 may comprise the semiconductor optic radiation source 102, the array 106 of single-photon avalanche diodes 400 and the data processing unit 110. By integrating the electronic circuits on the single circuit board the optoelectronic apparatus can be made wearable. In in embodiment, the weight of the optoelectronic apparatus can be kept at or below 100 g by also properly selecting the packaging and case material. In in embodiment, the weight of the optoelectronic apparatus can be kept at or below 50 g by properly selecting the packaging and case material. In in embodiment, the weight of the optoelectronic apparatus can be kept at or below 10 g.

In the manner explained, it is possible to form a histogram based on timings of detections of photons scattered from various and/or determined depths of the tissue. Namely, the depth can be determined by selecting or setting a suitable time-window. From these histograms i.e. digital time-of-flights it is possible to separate several bio-signals and/or unintended movement (disturbance) of the tissue which are mixed together but which appear and affect differently in different depths. Because of the differences depending of depths the separation is possible.Figure 14 is a flow chart of the measurement method. In step 1200, infrared pulses is output repeatedly toward the tissue by a semiconductor optic radiation source 102.

In step 1202, photons of the optical pulses that have interacted with the tissue are detected by an array 106 of single-photon avalanche diodes 400 which is directed toward the tissue.

In step 1204, time-of-flights of photons of each of the optical pulses within a temporal measurement range after an output of each of the optical pulses are determined by a timing unit 108.

In step 1206 a physiological state at at least one unique depth range within the tissue is estimated using detected photons that have interacted with the tissue inside at least one time window 504, 506, 508, w1 to wn shorter than the temporal measurement range 510, by a data processing unit 110, where each of the at least one time window (504, 506, 508, w1 to wn) corresponds to a unique depth range (d1 to dn) within the tissue, the estimation being based on at least one of the following: a number of the detections within the at least one time window 504, 506, 508, w1 to wn and a distribution of the detections within the at least one time window 504, 506, 508, w1 to wn.

The method of estimating a physiological state of the tissue of Figure 13 may be implemented as a logic circuit solution or computer program. The computer program may be placed on a computer program distribution means for the distribution thereof. The computer program distribution means is readable by a data processing device, and it encodes the computer program commands, carries out the measurements and optionally controls the processes on the basis of the measurements.

The computer program may be distributed using a distribution medium which may be any medium readable by the controller. The medium may be a program storage medium, a memory, a software distribution package, or a compressed software package. In some cases, the distribution may be performed using at least one of the following: a near field communication signal, a short distance signal, and a telecommunications signal.

It will be obvious to a person skilled in the art that, as technology advances, the inventive concept can be implemented in various ways. The invention and its embodiments are not limited to the example embodiments described above but may vary within the scope of the claims.

## Claims

1. An optoelectronic apparatus for measuring organic tissue configured to be attached with the organic tissue comprising:
a semiconductor optic radiation source (102) configured to output repeatedly infrared pulses toward the tissue;
an array (106) of single-photon avalanche diodes (400) directed toward the tissue and configured to detect at least one of the following of photons of the optical pulses that have interacted with the tissue: a number of detections of at least one wavelength dominantly absorbed by blood with deoxidized hemoglobin and a number of detections of at least one wavelength dominantly absorbed by blood with oxidized hemoglobin;
a timing unit (108) configured to determine time-of-flights of photons of each of the optical pulses within a temporal measurement range (150) after an output of each of the optical pulses, the timing unit (108) comprising a plurality of time-to-digital converters configured to measure arrival-times of detected photons, the temporal measurement range (150) being based on time-to-digital conversion by the time-to-digital converters of the timing unit (108);
a data processing unit (110) configured to:
reconstruct a distribution of the detections within at least one time window (504, 506, 508) of the temporal measurement range (150) and estimate a physiological state at at least one unique depth range within the tissue using detected photons that have interacted with the tissue inside at least one time window (504, 506, 508, w1 to wn) shorter than the temporal measurement range (510), enabling a layered measurement of the tissue, where each time window (504, 506, 508, w1 to wn) corresponds to a unique depth range (d1 to dn) within the tissue, the estimation being based on at least one of: a number of the detections within each time window (504, 506, 508, w1 to wn) and a distribution of the detections of a plurality of time windows (504, 506, 508, w1 to wn), and **characterized in that** said data processing unit is further configured to:
estimate a physiological state related to lactic acid based on at least one of the following: the number of detections of the at least one wavelength dominantly absorbed by the blood with oxidized hemoglobin, and a difference between the number of detections of the at least one wavelength dominantly absorbed by the blood with deoxidized hemoglobin and the number of detections of the at least one wavelength dominantly absorbed by the blood with oxidized hemoglobin.

2. The apparatus of claim 1, wherein each of the single-photon avalanche diodes (400) is configured to detect a single photon of each of the optical pulses that is scattered from the tissue toward the array (106).

3. The apparatus of claim 1, wherein the data processing unit (110) is configured to perform the estimation of the physiological state of the tissue within each of a plurality of the time windows (504, 506, 508) as a function of time, and separate and determine pulsation of heart based on the estimation.

4. The apparatus of claim 1, wherein at least one of the timing unit (108) and data processing unit (110) is programmable such that at least one of the temporal measurement range and the at least one time window (504, 506, 508) is repeatedly adjustable.

5. The apparatus of claim 1, wherein the semiconductor optic radiation source (102) and the array (106) of single-photon avalanche diodes (400) are spaced apart by a non-zero distance.

6. The apparatus of claim 1, wherein an output section (310) of the semiconductor optic radiation source (102) and an input section (310) of the array (106) of single-photon avalanche diodes (400) are optically coaxial.

7. The apparatus of claim 1, wherein the optoelectronic apparatus comprises a muscle measurement device (600), which is attached to a muscular area of a body (12) and comprises a radio transmitter (610), the semiconductor optic radiation source (102), the array (106) of single-photon avalanche diodes (400) and the timing unit (108), and a separate wearable device (602), which comprises a radio receiver (612) and the data processing unit (110); and the radio transmitter (610) is configured to transmit information on detections to the radio receiver (612), which is configured to feed the information to the data processing unit (110) for determining the physiological state of the tissue at the muscular area of the body (12).

8. The apparatus of claim 7, wherein the separate wearable device (602) comprises an additional semiconductor optic radiation source (102'), and an additional array (106') of the single-photon avalanche diodes (400) for determining the physiological state of the tissue.

9. The apparatus of claim 1, wherein the data processing unit (110) comprises one or more processors (900), and one or more memories (902) including computer program code; and
the one or more memories (902) and the computer program code configured to, with the one or more processors (900), cause the data processing unit (110) at least to estimate the physiological state of the tissue.

10. A method of measuring organic tissue, the method comprising
outputting (1200) repeatedly, by a semiconductor optic radiation source (102), infrared pulses toward the tissue;
detecting (1202), by an array (106) of single-photon avalanche diodes (400) which is directed toward the tissue, at least one of the following of photons of the optical pulses that have interacted with the tissue: a number of detections of at least one wavelength dominantly absorbed by blood with deoxidized hemoglobin and a number of detections of at least one wavelength dominantly absorbed by blood with oxidized hemoglobin;
determining (1204), by a timing unit (108) comprising a plurality of time-to-digital converters, time-of-flights of photons of each of the optical pulses within a temporal measurement range after an output of each of the optical pulses, the temporal measurement range (150) of the timing unit (108) being based on the time-to-digital converters which measure arrival-times of detected photons;
reconstructing, by a data processing unit (110), a distribution of the detections within at least one time window (504, 506, 508) of the temporal measurement range (150) and estimating (1206) a physiological state at at least one unique depth range within the tissue using detected photons that have interacted with the tissue inside at least one time window (504, 506, 508, w1 to wn) shorter than the temporal measurement range (510) by a data processing unit (110) for enabling a layered measurement of the tissue, where each time window (504, 506, 508, w1 to wn) corresponds to a unique depth range (d1 to dn) within the tissue, the estimation being based on one of: a number of the detections within the at least one time window (504, 506, 508, w1 to wn) and a distribution of the detections within the at least one time window (504, 506, 508, w1 to wn); and **characterized in that** said method further comprises: estimating, by the data processing unit (110), a physiological state related to lactic acid based on at least one of the following: the number of detections of the at least one wavelength dominantly absorbed by the blood with oxidized hemoglobin, and a difference between the number of detections of the at least one wavelength dominantly absorbed by the blood with deoxidized hemoglobin and the number of detections of the at least one wavelength dominantly absorbed by the blood with oxidized hemoglobin.

11. The method of claim 10, wherein wherein the data processing unit (110) further performs an estimation of the physiological state of the tissue within each of a plurality of the time windows (504, 506, 508) as a function of time, and
separating and determining pulsation of the heart based on the estimation.

## Patentansprüche

1. Optoelektronische Einrichtung zum Messen von organischem Gewebe, die zur Befestigung an dem organischen Gewebe ausgebildet ist, umfassend:
eine halbleiteroptische Strahlungsquelle (102), die ausgebildet ist, wiederholt Infrarotpulse in Richtung des Gewebes auszugeben;
ein Array (106) von Einzelphotonen-Lawinendioden (400), das auf das Gewebe gerichtet und ausgebildet ist, mindestens eines der folgenden von Photonen der optischen Pulse, die mit dem Gewebe wechselgewirkt haben, zu detektieren: eine Anzahl von Detektionen von mindestens einer Wellenlänge, die von Blut mit deoxidiertem Hämoglobin vorwiegend absorbiert wird, und eine Anzahl von Detektionen von mindestens einer Wellenlänge, die von Blut mit oxidiertem Hämoglobin vorwiegend absorbiert wird;
eine Zeitmesseinheit (108), die ausgebildet ist, Laufzeiten von Photonen jedes der optischen Pulse innerhalb eines zeitlichen Messbereichs (150) nach einer Ausgabe jedes der optischen Pulse zu bestimmen, wobei die Zeitmesseinheit (108) eine Mehrzahl von Zeit/Digital-Wandlern umfasst, die ausgebildet sind, Ankunftszeiten detektierter Photonen zu messen, wobei der zeitliche Messbereich (150) auf der Grundlage von Zeit/Digital-Wandlung durch die Zeit/Digital-Wandler der Zeitmesseinheit (108) basiert;
eine Datenverarbeitungseinheit (110), die ausgebildet ist, Folgendes auszuführen:
eine Verteilung der Detektionen innerhalb von mindestens einem Zeitfenster (504, 506, 508) des zeitlichen Messbereichs (150) zu rekonstruieren und einen physiologischen Zustand bei mindestens einem eindeutigen Tiefenbereich innerhalb des Gewebes unter Verwendung detektierter Photonen, die innerhalb von mindestens einem Zeitfenster (504, 506, 508, w1 bis wn), das kürzer als der zeitliche Messbereich (510) ist, mit dem Gewebe wechselgewirkt haben, zu schätzen, wodurch eine schichtweise Messung des Gewebes ermöglicht wird, wobei jedes Zeitfenster (504, 506, 508, w1 bis wn) einem eindeutigen Tiefenbereich (d1 bis dn) innerhalb des Gewebes entspricht, wobei die Schätzung auf mindestens einem der folgenden beruht: einer Anzahl der Detektionen innerhalb jedes Zeitfensters (504, 506, 508, w1 bis wn) und einer Verteilung der Detektionen einer Mehrzahl von Zeitfenstern (504, 506, 508, w1 bis wn), und
**dadurch gekennzeichnet, dass** die Datenverarbeitungseinheit weiter ausgebildet ist, Folgendes auszuführen:
einen mit Milchsäure zusammenhängenden physiologischen Zustand auf der Grundlage von mindestens einem der folgenden zu schätzen: der Anzahl von Detektionen der mindestens einen Wellenlänge, die von dem Blut mit oxidiertem Hämoglobin vorwiegend absorbiert wird, und einer Differenz zwischen der Anzahl von Detektionen der mindestens einen Wellenlänge, die von dem Blut mit deoxidiertem Hämoglobin vorwiegend absorbiert wird, und der Anzahl von Detektionen der mindestens einen Wellenlänge, die von dem Blut mit oxidiertem Hämoglobin vorwiegend absorbiert wird.

2. Einrichtung nach Anspruch 1, wobei jede der Einzelphotonen-Lawinendioden (400) ausgebildet ist, ein einzelnes Photon jedes der optischen Pulse zu detektieren, das aus dem Gewebe in Richtung des Arrays (106) gestreut wird.

3. Einrichtung nach Anspruch 1, wobei die Datenverarbeitungseinheit (110) ausgebildet ist, die Schätzung des physiologischen Zustands des Gewebes innerhalb jedes einer Mehrzahl der Zeitfenster (504, 506, 508) als eine Funktion der Zeit durchzuführen und eine Pulsation des Herzens auf der Grundlage der Schätzung zu trennen und zu bestimmen.

4. Einrichtung nach Anspruch 1, wobei mindestens eine von der Zeitmesseinheit (108) und der Datenverarbeitungseinheit (110) programmierbar ist, so dass mindestens einer von dem zeitlichen Messbereich und dem mindestens einen Zeitfenster (504, 506, 508) wiederholt einstellbar ist.

5. Einrichtung nach Anspruch 1, wobei die halbleiteroptische Strahlungsquelle (102) und das Array (106) von Einzelphotonen-Lawinendioden (400) durch einen von Null verschiedenen Abstand beabstandet sind.

6. Einrichtung nach Anspruch 1, wobei ein Ausgangsabschnitt (310) der halbleiteroptischen Strahlungsquelle (102) und ein Eingangsabschnitt (310) des Arrays (106) von Einzelphotonen-Lawinendioden (400) optisch koaxial sind.

7. Einrichtung nach Anspruch 1, wobei die optoelektronische Einrichtung eine Muskelmessvorrichtung (600) umfasst, die an einem Muskelbereich eines Körpers (12) befestigt ist und einen Funksender (610), die halbleiteroptische Strahlungsquelle (102), das Array (106) von Einzelphotonen-Lawinendioden (400) und die Zeitmesseinheit (108) umfasst, und eine separate tragbare Vorrichtung (602), die einen Funkempfänger (612) und die Datenverarbeitungseinheit (110) umfasst; und wobei der Funksender (610) ausgebildet ist, Informationen über Detektionen an den Funkempfänger (612) zu übertragen, der ausgebildet ist, die Informationen der Datenverarbeitungseinheit (110) zum Bestimmen des physiologischen Zustands des Gewebes an dem Muskelbereich des Körpers (12) zuzuführen.

8. Einrichtung nach Anspruch 7, wobei die separate tragbare Vorrichtung (602) eine zusätzliche halbleiteroptische Strahlungsquelle (102') und ein zusätzliches Array (106') der Einzelphotonen-Lawinendioden (400) zum Bestimmen des physiologischen Zustands des Gewebes umfasst.

9. Einrichtung nach Anspruch 1, wobei die Datenverarbeitungseinheit (110) einen oder mehrere Prozessoren (900) und einen oder mehrere Speicher (902), die Computerprogrammcode einschließen, umfasst; und
wobei die einen oder mehreren Speicher (902) und der Computerprogrammcode ausgebildet sind, zusammen mit dem einen oder den mehreren Prozessoren (900) zu bewirken, dass die Datenverarbeitungseinheit (110) mindestens den physiologischen Zustand des Gewebes schätzt.

10. Verfahren zum Messen von organischem Gewebe, wobei das Verfahren Folgendes umfasst
wiederholtes Ausgeben (1200) von Infrarotpulsen in Richtung des Gewebes durch eine halbleiteroptische Strahlungsquelle (102);
Detektieren (1202) mindestens eines der Folgenden von Photonen der optischen Pulse, die mit dem Gewebe wechselgewirkt haben, durch ein Array (106) von Einzelphotonen-Lawinendioden (400), das auf das Gewebe gerichtet ist: eine Anzahl von Detektionen von mindestens einer Wellenlänge, die von Blut mit deoxidiertem Hämoglobin vorwiegend absorbiert wird, und eine Anzahl von Detektionen von mindestens einer Wellenlänge, die von Blut mit oxidiertem Hämoglobin vorwiegend absorbiert wird;
Bestimmen (1204) von Laufzeiten von Photonen jedes der optischen Pulse innerhalb eines zeitlichen Messbereichs nach einer Ausgabe jedes der optischen Pulse durch eine Zeitmesseinheit (108), die eine Mehrzahl von Zeit/Digital-Wandlern umfasst, wobei der zeitliche Messbereich (150) der Zeitmesseinheit (108) auf den Zeit/Digital-Wandlern beruht, die Ankunftszeiten detektierter Photonen messen;
Rekonstruieren, durch eine Datenverarbeitungseinheit (110), einer Verteilung der Detektionen innerhalb von mindestens einem Zeitfenster (504, 506, 508) des zeitlichen Messbereichs (150) und Schätzen (1206) eines physiologischen Zustands bei mindestens einem eindeutigen Tiefenbereich innerhalb des Gewebes unter Verwendung detektierter Photonen, die innerhalb von mindestens einem Zeitfenster (504, 506, 508, w1 bis wn), das kürzer als der zeitliche Messbereich (510) ist, mit dem Gewebe wechselgewirkt haben, durch eine Datenverarbeitungseinheit (110), zum Ermöglichen einer schichtweisen Messung des Gewebes, wobei jedes Zeitfenster (504, 506, 508, w1 bis wn) einem eindeutigen Tiefenbereich (d1 bis dn) innerhalb des Gewebes entspricht, wobei die Schätzung auf einem der folgenden beruht: einer Anzahl der Detektionen innerhalb des mindestens einen Zeitfensters (504, 506, 508, w1 bis wn) und einer Verteilung der Detektionen innerhalb des mindestens einen Zeitfensters (504, 506, 508, w1 bis wn); und
**dadurch gekennzeichnet, dass** das Verfahren weiter Folgendes umfasst:
Schätzen eines mit Milchsäure zusammenhängenden physiologischen Zustands auf der Grundlage von mindestens einem der folgenden durch die Datenverarbeitungseinheit (110): der Anzahl von Detektionen der mindestens einen Wellenlänge, die von dem Blut mit oxidiertem Hämoglobin vorwiegend absorbiert wird, und einer Differenz zwischen der Anzahl von Detektionen der mindestens einen Wellenlänge, die von dem Blut mit deoxidiertem Hämoglobin vorwiegend absorbiert wird, und der Anzahl von Detektionen der mindestens einen Wellenlänge, die von dem Blut mit oxidiertem Hämoglobin vorwiegend absorbiert wird.

11. Verfahren nach Anspruch 10, wobei wobei die Datenverarbeitungseinheit (110) weiter eine Schätzung des physiologischen Zustands des Gewebes innerhalb jedes einer Mehrzahl der Zeitfenster (504, 506, 508) als eine Funktion der Zeit durchführt, und
Trennen und Bestimmen einer Pulsation des Herzens auf der Grundlage der Schätzung.

## Revendications

1. Appareil optoélectronique de mesure de tissu organique, configuré pour être fixé au tissu organique, comprenant :
une source de rayonnement optique (102) à semi-conducteur, configurée pour émettre de façon répétée des impulsions infrarouges vers le tissu ;
un réseau (106) de diodes à avalanche monophotoniques (400), dirigé vers le tissu et configuré pour détecter au moins un des éléments suivants parmi des photons des impulsions optiques qui ont interagi avec le tissu : un nombre de détections d'au moins une longueur d'onde absorbée de manière prédominante par du sang contenant de l'hémoglobine désoxygénée et un nombre de détections d'au moins une longueur d'onde absorbée de manière prédominante par du sang contenant de l'hémoglobine oxygénée ;
une unité de chronométrage (108), configurée pour déterminer des temps de vol des photons de chacune des impulsions optiques dans une plage temporelle de mesure (150) après une émission de chacune des impulsions optiques, l'unité de chronométrage (108) comprenant une pluralité de convertisseurs temps-numériques configurés pour mesurer les temps d'arrivée des photons détectés, la plage temporelle de mesure (150) étant basée sur une conversion temps-numérique par les convertisseurs temps-numériques de l'unité de chronométrage (108) ;
une unité (110) de traitement de données, configurée pour :
reconstruire une distribution des détections dans au moins une fenêtre temporelle (504, 506, 508) de la plage temporelle de mesure (150) et estimer un état physiologique à au moins une plage de profondeur unique dans le tissu en utilisant des photons détectés qui ont interagi avec le tissu à l'intérieur d'au moins une fenêtre temporelle (504, 506, 508, w1 à wn) plus courte que la plage temporelle de mesure (510), permettant une mesure du tissu par couches, où chaque fenêtre temporelle (504, 506, 508, w1 à wn) correspond à une plage de profondeur unique (d1 à dn) dans le tissu, l'estimation étant basée sur au moins un parmi : un nombre des détections dans chaque fenêtre temporelle (504, 506, 508, w1 à wn) et une distribution des détections d'une pluralité de fenêtres temporelles (504, 506, 508, w1 à wn), et
**caractérisé en ce que** ladite unité de traitement de données est en outre configurée pour :
estimer un état physiologique lié à l'acide lactique sur la base d'au moins un parmi les éléments suivants : le nombre de détections de ladite au moins une longueur d'onde absorbée de manière prédominante par le sang contenant de l'hémoglobine oxygénée, et une différence entre le nombre de détections de ladite au moins une longueur d'onde absorbée de manière prédominante par le sang contenant de l'hémoglobine désoxygénée et le nombre de détections de ladite au moins une longueur d'onde absorbée de manière prédominante par le sang contenant de l'hémoglobine oxygénée.

2. Appareil selon la revendication 1, dans lequel chacune des diodes à avalanche monophotoniques (400) est configurée pour détecter un seul photon de chacune des impulsions optiques qui est diffusé depuis le tissu vers le réseau (106).

3. Appareil selon la revendication 1, dans lequel l'unité (110) de traitement de données est configurée pour effectuer l'estimation de l'état physiologique du tissu dans chaque fenêtre temporelle d'une pluralité des fenêtres temporelles (504, 506, 508) en fonction du temps, et séparer et déterminer la pulsation du cœur sur la base de l'estimation.

4. Appareil selon la revendication 1, dans lequel au moins une parmi l'unité de chronométrage (108) et l'unité (110) de traitement de données est programmable de sorte qu'au moins une parmi la plage temporelle de mesure et ladite au moins une fenêtre temporelle (504, 506, 508) est réglable de façon répétée.

5. Appareil selon la revendication 1, dans lequel la source de rayonnement optique (102) à semi-conducteur et le réseau (106) de diodes à avalanche monophotoniques (400) sont espacés par une distance non nulle.

6. Appareil selon la revendication 1, dans lequel une section de sortie (310) de la source de rayonnement optique (102) à semi-conducteur et une section d'entrée (310) du réseau (106) de diodes à avalanche monophotoniques (400) sont optiquement coaxiales.

7. Appareil selon la revendication 1, dans lequel l'appareil optoélectronique comprend un dispositif (600) de mesure musculaire, qui est fixé à une zone musculaire d'un corps (12) et comprend un émetteur radio (610), la source de rayonnement optique (102) à semi-conducteur, le réseau (106) de diodes à avalanche monophotoniques (400) et l'unité de chronométrage (108), et un dispositif portable séparé (602), qui comprend un récepteur radio (612) et l'unité (110) de traitement de données ; et l'émetteur radio (610) est configuré pour transmettre des informations sur des détections au récepteur radio (612), qui est configuré pour alimenter l'unité (110) de traitement de données avec les informations pour déterminer l'état physiologique du tissu au niveau de la zone musculaire du corps (12).

8. Appareil selon la revendication 7, dans lequel le dispositif portable séparé (602) comprend une source de rayonnement optique à semi-conducteur (102') supplémentaire, et un réseau supplémentaire (106') de diodes à avalanche monophotoniques (400) pour déterminer l'état physiologique du tissu.

9. Appareil selon la revendication 1, dans lequel l'unité (110) de traitement de données comprend un ou plusieurs processeurs (900), et une ou plusieurs mémoires (902) incluant du code de programme informatique ; et
les une ou plusieurs mémoires (902) et le code de programme informatique configurés pour, avec les un ou plusieurs processeurs (900), amener l'unité (110) de traitement de données au moins à estimer l'état physiologique du tissu.

10. Procédé de mesure de tissu organique, le procédé comprenant
l'émission (1200) de façon répétée, par une source de rayonnement optique (102) à semi-conducteur, d'impulsions infrarouges vers le tissu ;
la détection (1202), par un réseau (106) de diodes à avalanche monophotoniques (400) qui est dirigé vers le tissu, d'au moins un des éléments suivants parmi des photons des impulsions optiques qui ont interagi avec le tissu : un nombre de détections d'au moins une longueur d'onde absorbée de manière prédominante par du sang contenant de l'hémoglobine désoxygénée et un nombre de détections d'au moins une longueur d'onde absorbée de manière prédominante par du sang contenant de l'hémoglobine oxygénée ;
la détermination (1204), par une unité de chronométrage (108) comprenant une pluralité de convertisseurs temps-numériques, de temps de vol des photons de chacune des impulsions optiques dans une plage temporelle de mesure après une émission de chacune des impulsions optiques, la plage temporelle de mesure (150) de l'unité de chronométrage (108) étant basée sur les convertisseurs temps-numériques qui mesurent les temps d'arrivée des photons détectés ;
la reconstruction, par une unité (110) de traitement de données, d'une distribution des détections dans au moins une fenêtre temporelle (504, 506, 508) de la plage temporelle de mesure (150) et l'estimation (1206) d'un état physiologique à au moins une plage de profondeur unique dans le tissu en utilisant des photons détectés qui ont interagi avec le tissu à l'intérieur d'au moins une fenêtre temporelle (504, 506, 508, w1 à wn) plus courte que la plage temporelle de mesure (510) par une unité (110) de traitement de données, pour permettre une mesure par couches du tissu, où chaque fenêtre temporelle (504, 506, 508, w1 à wn) correspond à une plage de profondeur unique (d1 à dn) dans le tissu, l'estimation étant basée sur un parmi : un nombre des détections dans ladite au moins une fenêtre temporelle (504, 506, 508, w1 à wn) et une distribution des détections dans ladite au moins une fenêtre temporelle (504, 506, 508, w1 à wn) ; et
**caractérisé en ce que** ledit procédé comprend en outre :
l'estimation, par l'unité (110) de traitement de données, d'un état physiologique lié à l'acide lactique sur la base d'au moins un parmi les éléments suivants : le nombre de détections de ladite au moins une longueur d'onde absorbée de manière prédominante par le sang contenant de l'hémoglobine oxygénée, et une différence entre le nombre de détections de ladite au moins une longueur d'onde absorbée de manière prédominante par le sang contenant de l'hémoglobine désoxygénée et le nombre de détections de ladite au moins une longueur d'onde absorbée de manière prédominante par le sang contenant de l'hémoglobine oxygénée.

11. Procédé selon la revendication 10, dans lequel dans lequel l'unité (110) de traitement de données effectue en outre une estimation de l'état physiologique du tissu dans chaque fenêtre temporelle d'une pluralité des fenêtres temporelles (504, 506, 508) en fonction du temps, et
la séparation et la détermination de la pulsation du cœur sur la base de l'estimation.
